Europäisches Patentamt

European Patent Office

Office européen des brevets

(19)

(11) Veröffentlichungsnummer: **0 229 271**
**B1**

(12) **EUROPÄISCHE PATENTSCHRIFT**

(45) Veröffentlichungstag der Patentschrift: **08.08.90**

(51) Int. Cl.⁵: **A 61 M 1/30**

(21) Anmeldenummer: **86116330.1**

(22) Anmeldetag: **25.11.86**

(54) Dialysevorrichtung.

(30) Priorität: **14.01.86 DE 3600793**

(43) Veröffentlichungstag der Anmeldung:
**22.07.87 Patentblatt 87/30**

(45) Bekanntmachung des Hinweises auf die
Patenterteilung:
**08.08.90 Patentblatt 90/32**

(84) Benannte Vertragsstaaten:
**BE DE ES FR GB IT NL SE**

(56) Entgegenhaltungen:
**EP-A-0 096 973**
**EP-A-0 148 319**
**DE-A-2 236 433**
**DE-A-2 636 290**

(73) Patentinhaber: **B. Braun-SSC AG**
**Gerliswilstrasse 74**
**CH-6020 Emmenbrücke (CH)**

(72) Erfinder: **Bock, Gerhard, Dipl.-Ing.**
**Schulstrasse 2**
**D-6431 Friedewald (DE)**
Erfinder: **Panse, Michael, Dipl.-Ing.**
**Werner-Forssmann-strasse 24**
**D-3588 Homberg (DE)**
Erfinder: **Eckel, Heinrich**
**Am Steg 3**
**D-6442 Rotenburg/F. 1 (DE)**
Erfinder: **Heitmeier, Rolf, Dipl.-Ing.**
**Goethestrasse 8**
**D-3507 Baunatal (DE)**
Erfinder: **Rath, Dieter**
**Franz-Gleim-Strasse 69**
**D-3508 Melsungen (DE)**

(74) Vertreter: **von Kreisler, Alek, Dipl.-Chem. et al**
**Deichmannhaus am Hauptbahnhof**
**D-5000 Köln 1 (DE)**

Courier Press, Leamington Spa, England.

## Beschreibung

Die Erfindung betrifft eine Dialysevorrichtung für die Einnadeldialyse.

Bei der Einnadel-Hämodialyse wird der Patient nur an einer einzigen Stelle mit einer Nadel punktiert. Durch einer Nadel hindurch wird in einer ersten Phase dem Patienten Blut entnommen und dem Dialysator zugeführt, während in einer zweiten Phase das Blut vom Dialysator zum Patienten zurückgeleitet wird. Eine Dialysevorrichtung der eingangs genannten Art (DE-A-22 36 433) weist ein geschlossenes System auf, bei dem die einzige einlumige Nadel abwechselnd mit dem Arterienzweig und dem Venenzweig verbunden wird, während der jeweils andere Zweig abgesperrt ist. In der ersten Phase ist der Venenzweig abgesperrt. Die pumpe pumpt Blut vom Patienten über den Arterienzweig in den Dialysator. Die erste Phase wird beendet, wenn der Druck am Blut-Ausgang des Dialysators einen oberen Grenzwert erreicht. In der nachfolgenden zweiten Phase wird die Arterienleitung gesperrt und die Venenleitung geöffnet. Das Blut fließt dann unter Druckentladung durch die Venenleitung und die Nadel in den Patienten zurück. Das bekannte System basiert darauf, daß am Dialysator zeitlich variierende Drücke auftreten, die für die Steuerung der Vorrichtung ausgenutzt werden. Druckveränderungen an der Dialysatormembran stören aber die Funktion des Dialysators erheblich, da sie die Ultrafiltrationsrate beeinflussen und eine bilanzierte Ultrafiltration durch die Dialysatormembran hindurch unmöglich machen. Außerdem kann es bei Blutflußstillstand an der Dialysatormembran infolge der weiterlaufenden Ultrafiltration zu einem Abfall des venösen Rücklaufdruckes unter den zulässigen Grenzwert kommen, so daß die entsprechende Alarmeinrichtung anspricht.

Bei einer weiteren bekannten Dialysevorrichtung (DE-A-33 35 744) ist die Expansionskammer in einen Parallelzweig zu der einzigen Pumpe geschaltet. Die Pumpe fördert in der ersten Phase das Blut in die Expansionskammer, während der Blutweg durch den Dialysator abgesperrt ist; in der zweiten Phase fördert die Pumpe bei abgesperrter Arterienleitung das Blut aus der Expansionskammer zum Dialysator. Auch hierbei ergeben sich starke Schwankungen des Transmembrandrucks an der Dialysatormembran und eine schrittweise unterbrochene, diskontinuierliche Dialyse. Die Aufrechterhaltung eines unteren Grenzwertes des venösen Rücklaufdruckes ist nicht ohne weiteres möglich.

Der Erfindung liegt die Aufgabe zugrunde, eine Dialysevorrichtung der eingangs genannten Art zu schaffen, bei der der von der Blutseite her auf die Membran des Dialysators einwirkende Druck bei abgesperrter Venenleitung keinen größeren Schwankungen unterworfen ist.

Bei der erfindungsgemäßen Dialysevorrichtung wird in der ersten Phase, also bei abgesperrter Venenleitung, dem Dialysator weiterhin Blut zugeführt, und zwar durch die zweite Pumpe, während die erste pumpe Blut aus der Nadel zur Expansionskammer fördert. Die zweite Pumpe ist dabei so angetrieben, daß sie die Ultrafiltrationsrate des Dialysators ausgleicht, d.h. diejenige Flüssigkeitsmenge ersetzt, die die Dialysatormembran passiert. Auf diese Weise wird der blutseitige Druck auf die Dialysatormembran im wesentlichen konstant gehalten, so daß eine Bilanzierung der Ultrafiltration möglich ist. Dies bedeutet, daß die Ultrafiltrationsmenge pro Zeiteinheit im wesentlichen konstant ist. Durch die Erfindungwird ferner sichergestellt, daß der venöse Rücklaufdruck in der erforderlichen Höhe gehalten wird. Ein etwaiger Schlauchbruch in der venösen Rücklaufleitung wird anhand des auftretenden Druckabfalls mit Sicherheit erkannt.

Gemäß einer bevorzugten Weiterbildung ist die zweite Pumpe bei abgesperrter Arterienleitung mit konstanter Geschwindigkeit angetrieben. Die zweite Pumpe fördert das in der Expansionskammer enthaltene Blut mit konstanter Förderrate durch den Dialysator. In dieser phase wird also die Blutförderung nicht von der ersten Pumpe übernommen und auch nicht durch Druckentspannung der Expansionskammer. Durch den gleichmäßigen Betrieb der zweiten Pumpe wird eine zeitlich konstante Blutzufuhr zum Dialysator in der zweiten Phase erreicht, während die erste Pumpe stillsteht und die Arterienleitung abgesperrt ist.

Vorzugsweise erfolgt die Regelung der Antriebsgeschwindigkeit der zweiten Pumpe durch einen an die Venenleitung angeschlossenen Drucksensor, dessen Ausgangssignal bei abgesperrter Venenleitung die zweite Pumpe steuert. Die Venenleitung kann ferner eine Luftfalle enthalten, deren Luftraum mit dem Drucksensor verbunden ist.

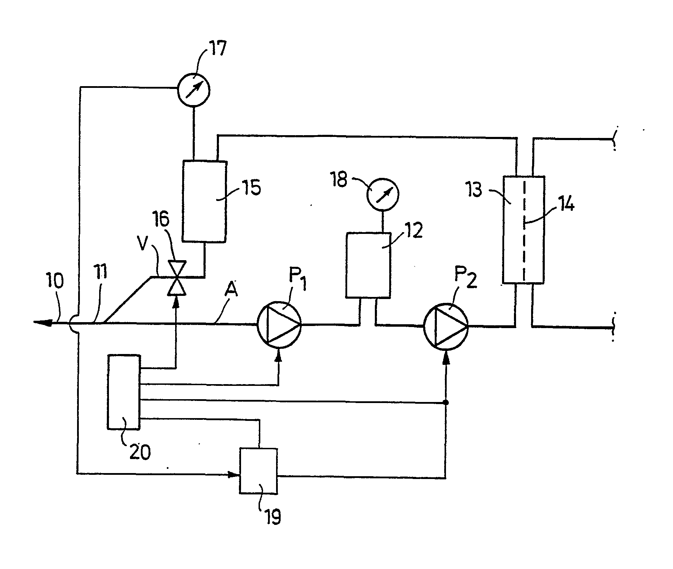

Im folgenden wird unter Bezugnahme auf die einzige Figur der Zeichnung ein Ausführungsbeispiel der Erfindung näher erläutert. In der Zeichnung ist der Aufbau der Dialysevorrichtung schematisch dargestellt.

Bei dem dargestellten Ausführungsbeispiel ist eine einlumige Nadel 10, deren Spitze in ein Blutgefäß des Patienten eingestochen wird, mit einer Blutleitung 11 verbunden. Die Blutleitung 11 verzweigt sich in eine Arterienleitung A und eine Venenleitung V. In der Arterienleitung A befinden sich hintereinander die Pumpe P1, die Expansionskammer 12, die Pumpe P2 und der Dialysator 13. Die Membran des Dialysators ist mit 14 bezeichnet.

Die Venenleitung V enthält das Absperrventil 16 und die luftfalle 15 und ist an den Blut-Auslaß des Dialysators 13 angeschlossen. Die Luftfalle 15 ist ein geschlossener Behälter, von dessem oberen Ende eine Druckleitung zu einem Drucksensor 17 führt, der zusätzlich als Anzeigegerät ausgebildet sein kann.

Die beiden Pumpen P1 und P2 sind Verdrängerpumpen, vorzugsweise peresstaltische Schlauchpumpen. Wenn die Pumpe P1 stillsteht, quetscht sie den Schlauch ab und wirkt somit als Absperrvorrichtung für die Arterienleitung A. Die Absperrvorrichtung der Venenleitung V wird von dem Absperrventil 16 gebildet.

Beim Betrieb der Dialysevorrichtung ist in der ersten Phase das Absperrventil 16 geschlossen und die erste Pumpe P1 fördert Blut vom Patienten in die Expansionskammer 12. Die Expansionskammer ist ebenfalls eine geschlossene Kammer, aus der bei steigendem Blutpegel die Luft verdrängt oder komprimiert wird. Der Druck in der Expansionskammer kann mit einem Druckmesser 18 angezeigt und ggf. zur Steuerung des Gerätes ausgewertet werden. Während Blut in die Expansionskammer 12 gefördert wird, pumpt die zweite Pumpe P2 Blut aus der Expansionskammer zum Blut-Einlaß des Dialysators. Die Förderrate der zweiten Pumpe wird von dem Drucksensor 17 gesteuert, der ein Signal an den Regler 19 zur Regelung der Pumpendrehzahl liefert. Die Pumpe P2 liefert an den Dialysator eine solche Blutmenge, daß der durch Ultrafiltration entstandene Flüssigkeitsverlust ausgeglichen wird und der Druck in der Luftfalle 15 konstant bleibt. Die erste Phase wird beendet, wenn der Druck in der Expansionskammer 12 einen vorgegebenen Grenzwert erreicht, oder wenn der Blutpegel einen vorgegebenen Grenzwert erreicht oder auch nach Ablauf einer vorbestimmten Zeitspanne.

Nach Beendigung der ersten Phase erfolgt die zweite Phase, d.h. die Phase des Rücklaufs des gereinigten Blutes zum Patienten. In dieser Phase steht die erste Pumpe P1 still und sperrt die Arterienleitung A ab, während das Absperrventil 16 der Venenleitung V geöffnet ist. Die zweite Pumpe P2 pumpt nun Blut aus der Expansionskammer 12 durch den Dialysator 13 zur Venenleitung V und zum Patienten. Die Antriebsgeschwindigkeit der pumpe P2 ist in dieser zweiten Phase konstant, kann jedoch verstellt werden. Die zweite Phase wird beendet, wenn der Blutpegel einen vorgegebenen unteren Grenzwert erreicht oder der Druck in der Expansionskammer 12 einen vorgegebenen unteren Grenzwert erreicht oder nach Ablauf einer vorbestimmten Zeitspanne.

Die beschriebenen Vorgänge werden durch das Steuergerät 20 gesteuert, das die Umschaltung des Absperrventils 16 und der Pumpe P1 sowie die Einschaltung der pumpe P2 in der zweiten phase vornimmt. Das Steuergerät 20 schaltet darüber hinaus nur in der ersten Phase den Regler 19 ein, so daß dieser die Drehzahl der pumpe P2 zur Konstanthaltung des Drucks in der Luftfalle 15 bzw. am Auslaß des Dialysators regeln kann.

**Patentansprüche**

1. Dialysevorrichtung für die Einnadeldialyse, mit

einer einzigen Nadel (10) zum Punktieren eines Blutgefässes,

einer an die Nadel (10) angeschlossenen Blutleitung (11), die sich in eine Arterienleitung (A) und eine Venenleitung (V) verzweigt,

einem die Arterienleitung mit der Venenleitung verbindenden Dialysator (13),

zwei abwechselnd betätigbaren Absperrvorrichtungen in der Arterienleitung und der Venenleitung,

einer ersten Pumpe (P1) in der Arterienleitung (A),

und einer zwischen den Auslaß der ersten Pumpe (P1) und den Blut-Einlaß des Dialysators geschalteten Expansionskammer (12),

ein Steuergerät (20) zur Steuerung der Pumpe und Absperrvorrichtungen,

dadurch gekennzeichnet, daß zwischen die Expansionskammern (12) und den Blut-Einlaß des Dialysators (13) eine zweite Pumpe (P2) geschaltet ist, und daß das Steuergerät (20) die zweite Pumpe (P2) bei geöffneter Arterienleitung (A) durch einen Regelmechanismus derart antreibt, daß sie den durch Ultrafiltration bewirkten Druckabfall in der abgesperrten Venenleitung im wesentlichen ausgleicht.

2. Dialysevorrichtung nach Anspruch 1, dadurch gekennzeichnet, daß die zweite Pumpe (P2) bei abgesperrter Arterienleitung (A) mit konstanter Geschwindigkeit angetrieben ist.

3. Dialysevorrichtung nach Anspruch 1 oder 2, dadurch gekennzeichnet, daß an die Venenleitung (V) ein Drucksensor (17) angeschlossen ist, dessen Ausgangssignal bei abgesperrter Venenleitung die zweite Pumpe (P2) steuert.

4. Dialysevorrichtung nach Anspruch 3, dadurch gekennzeichnet, daß die Venenleitung (V) eine Luftfalle (15) enthält, deren Luftraum mit dem Drucksensor (17) verbunden ist.

**Revendications**

1. Dispositif de dialyse pour la dialyse à une seule aiguille, comportant

une seule aiguille (10) destinée à être enfoncée dans un vaisseau sanguin,

une conduite de sang (11) raccordée à l'aiguille (10) et se subdivisant en une conduite artérielle (A) et en une conduite veineuse (V),

un dialyseur (13) reliant la conduite artérielle à la conduite veineuse,

deux dispositifs d'isolement dans la conduite artérielle et dans la conduite veineuse, pouvant être déclenchés alternativement,

une première pompe (P1) dans la conduite artérielle (A),

et une chambre de détente (12) montée entre la sortie de la première pompe (P1) et l'entrée de sang du dialyseur,

un appareil de commande (20) destiné à piloter la pompe et les dispositifs d'isolement,

dispositif caractérisé en ce qu'une deuxième pompe (P2) est montée entre la chambre de détente (12) et l'entrée de sang du dialyseur (13), et en ce que l'appareil de commande (20) entraîne, lorsque la conduite artérielle est ouverte, la deuxième pompe (P2) au moyen d'un mécanisme de régulation, de telle sorte qu'elle compense sensiblement la chute de pression occasionnée par l'ultrafiltration dans la conduite

veineuse fermée.

2. Dispositif de dialyse selon la revendication 1, caractérisé en ce que la deuxième pompe (P2) est entraînée à vitesse constante lorsque la conduite artérielle (A) est fermée.

3. Dispositif de dialyse selon la revendication 1 ou 2, caractérisé en ce que à la conduite veineuse (V) est relié un capteur de pression (17) dont le signal de sortie pilote la deuxième pompe (P2) lorsque la conduite veineuse est fermée.

4. Dispositif de dialyse selon la revendication 3, caractérisé en ce que la conduite veineuse (V) renferme un purgeur d'air (15) dont l'enceinte d'air est reliée au capteur de pression (17).

## Claims

1. Dialytic apparatus for single-needle dialysis, comprising

one single needle (10) for punctuating a blood vessel;

a blood conduit (11) connected to the needle (10) and branching into an artery conduit (A) and a vein conduit (V);

a dialyzer (13) connecting the artery conduit with the vein conduit;

two shut-off devices in the artery conduit and the vein conduit which shut-off devices can be actuated alternately;

a first pump (P1) in the artery conduit (A);

and an expansion chamber (12) interposed between the outlet port of the first pump (P1) and the blood-inlet port of the dialyzer, and

a control device (20) for controlling the pump and the shut-off devices

characterized in that a second pump (P2) is interposed between the expansion chamber (12) and the blood-inlet port of the dialzyer (13), and that said control device (20) drives said second pump (P2) when the artery conduit (A) is open by a regulating means in such a manner that the pressure decrease in the shut-off vein conduit which is caused by ultrafiltration, is substantially counterbalanced.

2. Dialytic apparatus according to claim 1, characterized in that the second pump (P2) is driven at a constant speed when the artery conduit (A) is shut off.

3. Dialytic apparatus according to claim 1, characterized in that a pressure sensor (17) is connected to the vein conduit (V), the output signal of the pressure sensor controlling the second pump (P2) when the vein conduit is shut off.

4. Dialytic apparatus according to claim 3, characterized in that the vein conduit (V) comprises an air trap (15) the air space of which is connected to the pressure sensor (17).